# EUROPEAN PATENT SPECIFICATION

(11) **EP 1 173 207 B1**
(45) Date of publication and mention of the grant of the patent: **06.11.2002**
(21) Application number: 00927713.8
(22) Date of filing: 14.04.2000
(51) Int. Cl.: A61K 45/06, A61P 27/02

(54) **MIOTIC AGENTS AND HYPERTONIC AGENTS CONTAINING OPHTHALMIC COMPOSITIONS**
MIOTISCHE WIRKSTOFFE UND HYPERTONISCHE WIRKSTOFFE ENTHALTENDE OPHTHALMISCHE ZUSAMMENSETZUNGEN
COMPOSITIONS OPHTALMIQUES CONTENANT DES AGENTS MIOTIQUES ET DES AGENTS HYPERTONIQUES

(30) Priority: 26.04.1999 IT RM990259
(43) Date of publication of application: 23.01.2002
(73) Proprietor: FARMIGEA S.P.A., I-56127 Pisa (IT)
(72) Inventor: Boldrini, Enrico, 56127 Pisa (IT); Severino, Dario Ercole, 56127 Pisa (IT); Panelli, Giorgio, 55100 Lucca (IT); Bianchini, Pietro, 56127 Pisa (IT)
(74) Representative: Banchetti, Marina
(86) International application number: IT0000151
(87) International publication number: WO00064425

(56) References cited:
- US-A- 4 590 207
- CONRAD, J. M. ET AL: "Influence of tonicity and pH on lacrimation and ocular drug bioavailability" J. PARENTER. DRUG ASSOC. (1978), 32(4), 149-61 , XP000911937
- MARISI A. ET AL: "Hypertonic saline solution in corneal edema." ANNALS OF OPHTHALMOLOGY, (1975) 7/2 (229-233). CODEN: ANOPB5, XP000912190
- Rote Liste, 1999, nos.: 68163-68182

## Description

The present invention relates to ophthalmic compositions for the treatment of visual disorders characterised by a reduced contrast sensitivity. More particularly, the invention relates to an association of active principles able to reduce Impairments of the visual function (like halos, glare and reduction of night and twilight vision) resulting from keratectomy operations carried out both with laser and by conventional techniques, from intraocular lens implantation (cataract surgery) and also resulting from various chronic pathologies affecting the anterior segment of the eyeball, such as the degenerations of the corneal tissue.

As known, ophthalmic surgery - particularly refractive surgery, which aims at modifying the refractive power of the eye in order to correct visual disabilities, usually of not minor seriousness - exploits a number of variously consolidated or developing surgical techniques, some examples of which are radial keratotomy, epikeratophakia and keratomileusis. In addition to these, a remarkable importance has been given, also i n the ophthalmology field, to the use of laser, particularly solid state laser, (like neodymium:yttrium-aluminum-garnet laser also known as Nd:YAG laser), and, above all, excimer laser.

Excimer laser is a pulsed laser which, due to the decay of noble gas dimers in the excited state (i.e. excimers, obtained from gas mixtures of halogens and noble gases), is able to emit large amounts of energy in the form of radiation in the range of far ultraviolet (UV-C), in pulse trains having predetermined duration, frequency and fluence. Each photon emitted during the irradiation has enough energy to break the intramolecular bonds of the exposed material, so that the irradiated molecules are "broken" in small volatile fragments which are expulsed at supersonic speed, resulting in a process known as "photodecomposition".

In the application of the excimer laser to corneal surgery procedures a type of argon-fluoride laser, emitting radiation with a wavelength of 193 nm is usually employed. Such type of laser turned out to be able to carry out highly precise operations with an optimal control on the penetration depth and a minimal thermal or mechanical damage effect on tissue adjacent to the exposed areas. Contrary to other lasers used in the clinical field, the excimer laser does not emit energy concentrated in a focal point but it has a beam with a large cross-section which, passing through suitable openings, is directed to strike large surface zones of the cornea, with an accurate control of the shape and size of the exposed zones. The emitted energy is almost totally absorbed by a surface layer a few microns thick and results in the ablation, by evaporation, of cornea layers little more thicker than molecular at every pulse, with a reproducibility not attainable by other techniques.

The excimer laser is widely used for refractive remodelling of the cornea by the techniques known as photorefractive keratectomy or PRK and LASIK (laser intrastromal keratomileusis), for the correction of various ametropias, among which the most diffused one is myopia. As it is known, the latter is a defect determined by a corneal curvature higher than required by the length of the eyeball, resulting in light rays from outside being refracted in a such way that they converge in a focal point before they reach the retina. In this circumstance the use of the excimer laser allows to ablate layers of corneal tissue having increasing thickness toward the centre of the treated area, thus reducing the curvature of the cornea. When the technique is used for the correction of hypermetropia, wherein, on the contrary, the modification to be obtained is an increase of the corneal curvature, the amount of ablated tissue at the periphery of the exposed zone is more important than in the centre. Finally, for the correction of astigmatism - which, as known, is an ametropia caused by irregular curvatures in the various meridians of the ocular surface - the depth of the ablated layers can be asymmetric, depending on the meridian to be "flattened".

In this connection, it should be noted that although the corneal epithelium is more responsive to the excimer laser than the underlying stroma, its response is different depending on whether the cell nucleus or the cytoplasm is considered, the nucleus being more resistant In view of that, the photorefractive technique known as PRK involves the mechanical ablation of the corneal epithelium (epithelial excision) before the actual tissue photodecomposition, so that the cornea can be treated in an even and quantifiable manner starting from the anterior surface of the stroma. On the other hand, the more recently developed LASIK technique involves cutting a corneal lamella with a predetermined thickness using a microkeratome (keratomileusis), lifting up the resulting lenticular flap (nasal or upper), photoablating by excimer laser the thus exposed portion of the underlying stroma and finally repositioning the lifted exterior corneal flap.

In addition to the photorefractive techniques, the excimer laser is also used for the therapeutic removal of surface corneal tissue, for the treatment of corneal irregularities and opacities of various nature, i.e. of dystrophic, degenerative, cicatricial or infective origin. Such surgical technique, called phototherapeutic keratectomy or PTK, has been used, for example, for the treatment of recurrent corneal erosions, post-operative keratites, corneal dystrophies such as Reis-Bückler dystrophy, corneal opacities or scars caused by Herpes simplex, surface irregularities following surgery, for example as outcomes of keratoplasty or refractive corneal operations. Contrary to refractive photokeratectomy, PTK aims at eliminating irregularities from the corneal surface in order to smooth the profile thereof, and therefore involves the ablation of tissue layers of different thickness in the various zones of the treated corneal surface.

Although the above described photokeratectomy procedures appear to be an alternative less traumatic than conventional techniques of ophthalmic surgery, the restorative process after photoablation is not free from drawbacks. These are variously transitory and bothering or disenabling for the patient, and include, for example, problems in corneal healing, generation of subepithelial opacities known as "haze" - which result in a reduction of the visual efficiency as a consequence of the "light scattering" phenomenon (light diffusion) - and, in some circumstances, regression of the refractive correction obtained with the operation, in addition to, of course, pain and risks of microbial infection which occur immediately after the operation. Actually, the restoration of the tissues involved in the irradiation occurs by means of a process which can be affected by both dinical and pharmacological factors in such a way that it is practically impossible to exactly predict the final optical correction obtainable.

Specifically, the morphological anomalies of the restorative process which occur most often include, firstly, epithelial hyperplasia. The latter represents a transitory, nearly physiological response which is substantially useful for the cicatrization, although its role in any refractive variations immediately after operation is not completely understood. A second, and more critical morphologic anomaly that may occur is the formation of subepithelial opacities (haze), the severity of which usually depends on the depth of the photoablation. Such complication principally occurs during the first months after the operation, and is followed by a progressive recovery. The said corneal opacities are probably due to microvacuoles or to intralamellar inclusions, to the presence of neoformed material (collagen III-IV, laminin, fibronectin, keratan sulfate, proteoglycans) and to irregularities in the junction between epithelium and stroma. In addition to the foregoing anomalies, comeal pachymetry studies carried out at 1-3 months following PRK or LASIK showed, for 30-40% of the cases, a corneal thickening resulting from stromal hydration (oedema). The latter is presumably determined by an acoustic shock wave, a phenomenon induced by the laser-tissue interaction, displacing water contained in the cell to the stroma. Finally, anomalies which can negatively affect the vision quality during the restorative process have also been evidenced by corneal topography studies. These studies allowed to detect irregularities within the structure of the epithelial layers, resulting from the migration and the hyperplasia of the corresponding cells.

The pharmacological therapy applied following photokeratectomy substantially consists in the topical ocular administration of antibiotics and antiinflammatory drugs. The use of antibiotics (mainly chloramphenicol and aminoglycosides like gentamicin and tobramycin, or fluoroquinolones like ofloxacin) is necessarily prolonged, after treatment by PRK, until the epithelium restoration is completed, so that the ocular surface deprived of the epithelium can be maintained aseptic. After treatment by LASIK, antibiotics are used until the cicatrization of the corneal lenticule with the surrounding tissue is completed. With respect to the antiinflammatory drugs, it is to be pointed out that the use of steroidal antiinflammatory drugs (like prednisolone, dexamethasone, fluorometholone, etc.) after photoablation is presently very debatable, whereas at first this therapy was considered to be the treatment of choice for the two main adverse effects of the laser surgery, that is the haze and, in case of medium and high myopia, the regression of the refractive correction obtained by the surgical intervention. The use of steroid drugs has caused in some circumstances one of the typical adverse effects resulting from the extended topical application of steroids, i.e. iatrogenic intraocular hypertension. Furthermore, the effectiveness of topical steroid drugs in normalising the stromal structure in the remodelling process of the corneal tissue after surgery was questioned as well. According to the above observations, topical non steroidal antiinflammatory drugs (NSAIDs), including diclofenac, indomethacin, ketorolac, etc., have been introduced as an alternative postoperative therapy. These drugs also allow to achieve a reduction of the pain resulting from the ablation of the corneal epithelium.

None of the pharmacological remedies considered for the treatment following photokeratectomy is specifically directed to the elimination or amelioration of the "visual disorders", reported in the postoperative follow-up, which are particularly evident under some specific illumination conditions (i.e., night vision, low light vision, high light vision). In this regard, several authors reported, for example, that the deterioration of night vision (i.e., scotopic vision) in patients treated by PRK is a quite usual occurrence, which negatively affects the living conditions of these patients over the period following the operation. For example, some papers (D.S. Gartry et al., *Photorefractive keratectomy with an argon fluoride excimer laser a clinical study,* Refract. Corneal Surg., 7:420-35, (1991); D.S. Gartry et al., *The effect of topical corticosteroids on refractive outcome and corneal haze after photorefractive keratectomy,* Arch. Ophthalmol., 110:944-52 (1992); D.P.S. O'Brart et al., *Night vision after excimer laser photorefractive keratectomy: haze and halos,* Eur. J. Ophthalmol., 4:43-61 (1994)) quantified the disorders of night vision at six months after PRK in terms of halos perceived around the light sources, as shown in the following table.

**TABLE 1**

| Quality of night vision and halos after PRK | | | |
|---|---|---|---|
| | Gartry et al. (1991) | Gantry et al. (1992) | O'Brart et al. (1997) |
| Total no. of patients. | 120 | 102 | 85 |
| Diameter of ablated zone (mm) | 4 | 4 | 5 |
| Halos around light sources at night (no. of patients (%)) | 97 (78%) | - | 38 (45%) |
| Halos causing disturbance in night vision (no. of patients (%)) | 12 (10%)* | 12 (12%) | 6 (7%) |
| Halos causing inability to nighttime driving (no. of patients (%)) | 12(10%)* | 15(15%) | 9(11%) |

| | | | |
|---|---|---|---|
| * in this study the two classes of patients were not considered distinct | | | |

It is to be pointed out that the symptoms referred to above can be perceived as being more or less critical depending on the living habits of the patients. Accordingly, the subjects which usually do not drive by night do not tend to consider the halos as a problem. Further, seasonal variations of daytime illumination can affect the severity of symptoms, and the patients for which the first postoperative period falls in winter are likely to suffer more seriously.

As mentioned above, another side effect of laser treatment which often results in transitory disorders of the visual function is the formation of corneal opacities or haze, which is mainly perceived as a dazzling effect around the light sources. This effect, which is particularly evident at low illumination levels (scotopic vision or mesopic, i.e. twilight, vision), can be particularly disenabling in the presence of more than one bright light source, as for example in a road illuminated by night.

For the evaluation of visual disorders resulting from refractive photokeratectomy treatments, as well as in various other cases involving a deterioration of the visual function, the study of the so-called "sensitivity to spatial contrast" has recently attracted a growing interest. The latter provides information about the overall spatial discriminating ability of the visual system. Although it is widely believed that the ability to recognise very small objects at long distance amounts to an optimum vision performance, it is not infrequent to find subjects who, while maintaining a good visual acuity - sometimes even better than 10/10 - report to suffer from not better defined "visual discomforts", mainly in special illumination conditions, said discomforts actually corresponding to a reduced contrast sensitivity.

In the study of contrast sensitivity, which can be defined as the ability to perceive small differences in brightness between light and dark bands separated from shaded boundaries, only the spatial frequency of the visual stimulus is varied, whereas the other two variables by which the said stimulus is characterised, i.e. its time frequency and its wavelength, are maintained unchanged. For the purpose of this study, the spatial frequency is defined as the number of pairs of light and dark bands subtended by a visual angle of one degree (1°) (such frequency being expressed in cycles per degree, cycles/°), and the contrast (C) is defined as the relative difference between maximum and minimum brightness (or luminance) of the considered bars. On this basis, the smallest contrast perceivable for each spatial frequency can be evaluated. Accordingly, the contrast sensitivity threshold (or threshold contrast) (S) is defined as the reciprocal of the minimum contrast which allows to perceive a predetermined spatial frequency (S = 1/C). By using standardised tables with different spatial frequency bands and contrast intensities, or by means of computer graphic systems, it is possible to obtain diagrams wherein the value of the minimum contrast perceived (or threshold contrast) is plotted as a function of the spatial frequency. As experimentally found out, in humans the maximum sensitivity corresponds to spatial frequencies of about 3 cycles/degree, while the highest perceivable spatial frequency is 50 cycles/degree.

One of the advantages of studying the sensitivity to spatial contrast rather than the visual acuity is due to the fact that the perception of low and medium spatial frequencies is not limited by the refractive properties of the eye, and also very wide gratings are perceived unchanged in the presence of refraction defects up to 15 diopters. This technique, sometimes in combination with the study of the contrast sensitivity by evoked potentials, has proved to be useful in ophthalmology in respect of several pathologies, and particularly in circumstances wherein the measure of visual acuity does not allow to draw an accurate picture as concerns the functional effects of the lesions. The said technique also plays a relevant role in the follow up of various ocular disorders affecting the anterior segment of the eyeball. Actually, it is unquestioned that glaucoma and abnormalities of the crystalline lens and of the cornea give rise, mainly in dazzlement conditions, to a reduction of the contrast sensitivity. Further, it has been proved that the postoperative follow-up for cataract and refractive surgery, for example carried out by excimer laser, can be suitably evaluated by this method.

Accordingly, in one of such recent works (S. Dutt et al., *One-year results of excimer laser photorefractive keratectomy for low to moderate myopia,* Arch. Ophthalmol., 112:1477-1436 (1994)) the postoperative follow-up in PRK interventions for the correction of myopia has been carried out by studying the contrast sensitivity, by means of determinations (for 47 eyes of 39 patients affected by myopia between -1.5 and -6 diopters) before and at six and twelve months after PRK. The data, shown in the following table, have been obtained by measuring (instrument Vector Vision CV-1000, Dayton, Ohio) the contrast sensitivity for each spatial frequency in cycles/degree both in conditions of pupillar dilatation and for not dilated pupil.

**TABLE 2**

| Contrast sensitivity before and after PRK* | | | | |
|---|---|---|---|---|
| | Spatial/frequencies, cycles/degree | | | |
| | 3 | 6 | 12 | 18 |
| Non-dilated pupil: | | | | |
| Preoperative | 5(1.2) | 4.4(1.2) | 3(1.4) | 2.9(1.3) |
| 6 months | 4.3(1.4) | 3.2(1.2) | 1.9(1.4) | 1.6(1.3) |
| 12 months | 4(1.1) | 3.4(1.3) | 1.7(1.3) | 2(1.3) |

| Dilated pupil: | | | | |
|---|---|---|---|---|
| Preoperative | 4(1.5) | 3.7(1.4) | 2.4(1.6) | 1.9(1.5) |
| 6 months | 3(1.3) | 2.4(1.5) | 1.5(1.3) | 1.4(1.3) |
| 12 months | 3.3(1.1) | 2.5(1.2) | 1.4(1.2) | 1.7(1.6) |

| | | | | |
|---|---|---|---|---|
| * values ( ) = +/- s.d. | | | | |

As it may be observed from the above table the postoperative values of contrast sensitivity are significantly lower than the preoperative ones, and this holds true for all spatial frequencies, both for dilated and for non-dilated pupil.

Another study, referred to below (J.D. Carr et al., *Prospective comparison of single-zone and multizone laser in situ keratomileusis for the correction of low myopia,* Ophthalmology, 105:1504-1511, (1998)) relates to the correction of myopia by LASIK. In this case subjects with myopia between -2.000 D and -7.000 D, who underwent either single zone (SZ) or multizone (MZ) photoablation, have been considered. The determination of the contrast sensitivity of the patients was carried out three months after the operation, and the relevant results are reported in the following table.

**TABLE 3**

| Contrast sensitivity three months after LASIK * | | | | | | | | |
|---|---|---|---|---|---|---|---|---|
| | 3 cycles/° | | 6 cycles/° | | 12 cycles/° | | 18 cycles/° | |
| | SZ | MZ | SZ | MZ | SZ | MZ | SZ | MZ |
| Non-dilated pupil: | | | | | | | | |
| Preoperative | 1.01 | 1.00 | 0.96 | 0.94 | 0.95 | 0.98 | 0.89 | 0.89 |
| Postoperative | 0.96^ | 0.95^ | 0.90^ | 0.91 | 0.92 | 0.89^ | 0.85 | 0.82 |

| Non-dilated pupil: | | | | | | | | |
|---|---|---|---|---|---|---|---|---|
| Preoperative | 0.94 | 0.94 | 0.90 | 0.91 | 0.86 | 0.87 | 0.73 | 0.73 |
| Postoperative | 0.86^ | 0.85 | 0.84^ | 0.83^ | 0.78^ | 0.76^ | 0.71 | 0.68 |

| | | | | | | | | |
|---|---|---|---|---|---|---|---|---|
| * s.d. values were omitted - mean values > 1 correspond to a better contrast sensitivity in comparison with normal myopic population | | | | | | | | |
| ^ p < 0.05 | | | | | | | | |

As it is shown by the table, three months after the operation by the LASIK technique a decrease in the contrast sensitivity has been detected at all four spatial frequencies considered, both for single zone and for multizone photoablation.

In view of the foregoing, it is an object of the present invention to provide a pharmacological treatment effective in eliminating, or at least remarkably ameliorating, the visual disorders characterised by a reduction of the contrast sensitivity which often result from refractive surgery (and namely following photokeratectomy using the PRK and LASIK techniques) and which are usually revealed by the perception of night halos or by dazzlement problems, as well as by a reduction of scotopic and mesopic vision.

Within the frame of the studies connected with this invention, it was considered that in both laser procedures referred to before the reshaped zones of the stroma, seen in rear projection, are larger than the pupil dilated in photopic vision; however, starting from the twilight, when the retina begins its scotopic activity and the pupil tends to have larger diameters, incident rays strike the periphery of the treated optical zone, resulting in diffractive phenomena (diffraction fringes along the edge of the treated zone). Furthermore, for the subjects having a pupillar diameter-larger than normal, the qualitative and quantitative worsening is also present in photopic vision.

On the other hand, and independently of the diffractive phenomena resulting from abnormalities in the stroma issue, the transparency of the cornea can be remarkably reduced by the corneal hydration resulting from the photodecomposition process. Actually, it is to be taken into account that corneal oedema induces a decrease of the corneal transparency, thus giving rise to a diffused haze. In the case of damage to the corneal endothelium, corneal oedema is accompanied by an increase of the tissue thickness and the proportion of water contained in the stroma, usually of about 78%, reaches values of about 85-90%.

The annoying diffractive phenomena mentioned before also occur in the follow-up of cataract surgery, until the eye becomes adapted to the intraocular lens implanted. As already pointed out, diffraction phenomena resulting in optic aberrations also occur in various chronic pathologies of the anterior segment of the eyeball, such as the degenerations of the corneal tissue.

On the basis of the foregoing considerations it has been found, according to the present invention, that a particular combination of drugs, applied on the ocular surface in the form of eye-drops, allows to effectively and readily restore the quality of the vision damaged following one of the above reported occurrences. Such combination, substantially consisting of a drug having miotic activity associated with a hypertonic (i.e. hyperosmolar) agent, presumably acts by the induction of a reduction of the pupillar diameter (caused by the miotic agent) which results in a stenopeic effect and, concurrently, by the restoration of the normal hydration state of the cornea (enhanced by the hypertonic agent), which tends to restore the transparency of the diopter. It is to be pointed out, however, that while the proposed combination is able to induce remarkable improvements in the contrast sensitivity measured using the above mentioned methods, each of the two proposed agents, when administered alone, does not appear to result in any appreciable improvement of the parameter considered herein as an index of the vision quality.

Both miotic agents and those having hyperosmolar activity are widely used for the therapy of ophthalmic affections. Specifically, miotic agents, and particularly parasympathomimetic or cholinomimetic (or cholinergic) agents, like pilocarpine, acetylcholine, aceclidine and carbachol, are active on the cholinergic receptors of the iris and of the ciliary muscles, causing the contraction of the sphincter muscle of the iris and the pupillary constriction (miosis), an increase of the tone and contraction of the ciliary muscles, with a relaxation of the zonule of Zinn and consequent increase of the curvature of the crystalline lens with spasm of accommodation for near vision (accomodation reflex). For example, the administration of pilocarpine eye-drops results in miosis within 30 minutes over a period of 4-8 hours and induces a decrease of the intraocular pressure within 75 minutes over a period of 4-14 hours, depending on the drug concentration employed.

Parasympathomimetic miotic agents are currently used in clinical medicine to reduce the intraocular pressure in the treatment of open angle glaucoma, often in combination with other topical anti-glaucoma drugs, like beta-blockers, adrenergic agents and carbonic anhydrase inhibitors. A further class of agents exhibiting miotic activity, also used in the treatment of glaucoma, are cholinesterase inhibitors, physostigmine being the most diffused member.

Both cholinergic agents and cholinesterase inhibitors, in their present application as anti-glaucoma drugs, are administered at concentrations usually between 0.25 and 10% by weight, the concentrations between 2 and 4% by weight being the most usual for pilocarpine and other cholinomimetic agents.

Hyperosmolar or hypertonic agents, which are used to reduce the corneal oedema for therapeutic or diagnostic purposes, act by exerting an osmotic attraction on water through the semi-permeable membrane of the corneal epithelium. Among these, the most frequently used agents are sodium chloride aqueous solutions at, e.g., 3 or 5% concentration by weight; glucose, normally as a solution at 40% by weight; glycerol, which however is used only in diagnostic preparations due to its short duration of action. A further hypertonic agent that has been proposed in the medical literature for the reduction of corneal oedema, is sodium sulfacetamide as a 30 wt% solution (M.N. Luxenberg and K. Green, Am. J. Ophthalmol., 71:847-853, (1971)), such preparation being already known and used in view of its antibacterial activity. Actually, it has been observed that a 30% aqueous solution of sodium sulfacetamide is remarkably hypertonic, having an osmolarity equivalent to that of a 9 wt.% solution of sodium chloride.

Although the use of sulfonamides as antibacterial agents has been mostly replaced by the new antibiotics, the topic ophthalmic use of sulfacetamide (in the form of the coresponding sodium salt, in solutions at 10-30% concentration by weight and as an ophthalmic ointment preparation at 10% concentration by weight) is still practised and reportedly effective for the prophylaxis of corneal infections resulting from epithelial abrasions or from the presence of foreign bodies, as well as for the treatment of developed infections caused by sensitive germs. In these situations sulfacetamide is normally instilled three times a day until the ocular surface is completely restored to its normal conditions. In some circumstances the ophthalmic use of the compound can result in a mild burning following the instillation; however, this initial irritation is usually followed by an analgesic effect resulting from a decrease of the corneal sensitivity. Such occurrence clearly appears to be advantageous, especially in cases where a corneal algesia deriving from epithelial alterations or abrasions is present. The instilled compound easily penetrates into the ocular tissues: within 5 minutes from the administration of a 30% solution the intraocular concentration of sodium sulfacetamide is already about 0.1%.

Although the invention is not specifically limited by any theoretical hypothesis about the mechanisms of action of the drugs in the association, it is believed that the proposed hypertonic ingredients are active on the corneal oedema both through a dehydration of the epithelium altered by the presence of vacuoles and through a reduction of the stromal swelling, deriving from an excessive water retention. Such events result in the restoration of the corneal diopter transparency, followed by a corresponding improvement of the vision quality. As already pointed out, however, the anti-oedema activity of the hyperosmotic preparation and the stenopeic effect of the miotic agent are combined in such a way that a synergistic effect is obtained from the proposed combination, while neither of the individual ingredients, used alone, appears to have any appreciable activity.

Therefore, the present invention specifically provides an ophthalmic composition for the treatment of visual disorders characterised by a reduced contrast sensitivity containing, in combination, therapeuticaliy effective amounts of one or more miotic agents selected from cholinomimetic agents and cholinesterase inhibitors, and a hypertonic amount of one or more agents active, when applied at hypertonic concentrations. In reducing corneal oedema. According to some specific embodiments of the invention, said oedema-reducing hypertonic agent is selected from sulfacetamide and derivatives thereof, sodium chloride, glucose and glycerol.

Preferably, the miotic agent is selected from pilocarpine and pharmaceutically acceptable acid addition salts thereof, carbachol, acetylcholine, aceclidine and phyisostigmine, while the hypertonic agent is sulfacetamide or a pharmaceutically acceptable salt thereof. Particularly preferred formulations indude, in combination with sulfacetamide as the hypertonic agent, pilocarpine or an add addition salt thereof at concentrations in the range from 0.05 to 5% by weight (expressed as the weight of the free base form of pilocarpine), the most preferred concentrations being remarkably lower than those currently used when said active ingredient is employed for the treatment of glaucoma, namely within the range from 0.05 to 0.5% by weight (again expressed as the free base form). Most suitably, the concentrations of sodium sulfacetamide are of the same order of magnitude as those employed in the administration of this agent in antmicrobial eye-drop preparations, i.e. within the range from 25 to 30% by weight, although any concentration ranging from 5 to 30% by weight may be conveniently employed.

The composition according to the invention can be prepared, generally, in the form of an aqueous solution or suspension for eye-drops or in the form of an emulsion, an ointment, a gel or a cream. Preferably, the product is administered in the form of an aqueous solution or suspension in a pharmaceutically acceptable ophthalmic vehicle. Obviously, the composition can include various excipients or adjuvants chosen among the ingredients usually employed in the pharmaceutical art for the formulation of topic ophthalmic preparations. Said excipients should be selected considering the stability characteristics of the resulting combination and the compatibility of said excipients with the two active ingredients. In particular, the composition should also comprise suitable preservatives and antimicrobial agents, such as, e.g., benzalkonium chloride, sodium merthiolate or thimerosal, methyl-, ethyl- and propyl paraben (i.e. methyl-, ethyl- and propyl-p-hydroxybenzoate), chlorobutonal, as well as chelating or sequestrating agents such as the edetates or EDTA, and antioxidants such as sodium metabisulfite.

When used in the form of an eye-drop preparation containing pilocarpine at concentrations in the range from 0.05 to 0.5% by weight and sodium sulfacetamide at concentrations in the range from 25 to 30% by weight, the preparation according to the invention can be administered, in order to obtain an appreciable reduction of the visual disorders resulting, e.g., from a photoablation treatment by excimer laser, at a dosage of one-two drops from two to three times a day, preferably two drops three times a day, starting a few days after surgery and continuing at least for two months, or in any case until the symptomatology has disappeared.

The present invention further provides the use of a combination of one or more miotic agents selected from cholinomimetic agents and cholinesterase inhibitors, and a hypertonic amount of one or more agents active, when applied at hypertonic concentrations, in reducing corneal oedema in the manufacture of a topical ophthalmic preparation for the treatment of visual disorders characterised by a reduced contrast sensitivity. Preferably, the said ophthalmic preparation has the optional features already referred to above, which features are recited in the dependent claims.

A specific embodiment of the drug combination according to the invention is described below, only by way of example, together with the results obtained from clinical tests carried out using the said combination, including a comparison with preparations containing, separately, each of said individual active ingredients.

### EXAMPLE

### Ophthalmic solution based on pilocarpine hydrochloride and sodium sulfacetamide

The preparation according to the invention, the performance of which was experimentally evaluated as it is briefly reported below, has the following composition:

| | | |
|---|---|---|
| pilocarpine hydrochloride | mg | 9.50 |
| (corresponding to ~ 8.1 mg of pilocarpine) | | |
| sodium sulfacetamide | g | 2.856 |
| metyl p-hydroxybenzoate | mg | 1.80 |
| propyl p-hydroxybenzoate | mg | 0.60 |
| sodium metabisulfite | mg | 28 |
| sodium edetate | mg | 5.60 |
| water | q.s. to ml | 10 |

The above ophthalmic solution, hereinafter referred to as "combination", underwent a series of studies and comparison tests aimed at evaluating the activity thereof in the treatment of visual disorders resulting from refractive photokeratectomy carried out by excimer laser, also in comparison with preparations containing only one of the two active ingredients. Specifically, the composition of the comparative preparation based on pilocarpine hydrochloride only, hereinafter referred to "pilocarpine", was as follows:

| | | |
|---|---|---|
| pilocarpin, hydrochloride | mg | 9.50 |
| (corresponding to ~ 8.1 mg of pilocarpine) | | |
| metyl p-hydroxybenzoate | mg | 1.80 |
| propyl p-hydroxybenzoate | mg | 0.60 |
| water | q.s. to ml | 10 |

The composition of the preparation based on sodium sulfacetamide only, referred to below as "sulfacetamide", was as follows:

| | | |
|---|---|---|
| sodium sulfacetamide | g | 2.856 |
| sodium metabilsulfide | mg | 28 |
| sodium edetate | mg | 5.60 |
| water | q.s. to ml | 10 |

### Clinical study

The investigation was carried out on a total of 110 patients (52.6% females and 47.4% males) who underwent refractive surgery by either the LASIK or the PRK technique.

The average age of the subjects included in the study was 31.3 ± 9.7; 50% of the patients were under 29 while 90% were not over 44. For the patients who underwent the LASIK treatment the diopter values were between -4 and -12 per eye, while for the patients subjected to PRK treatment the diopter values were between -1 and -3.75. Additional selection criteria for inclusion in the sample group, such as absence of developed ocular or systemic diseases, absence of topical or systemic therapies or of pregnancy, were satisfied too. Furthermore, the pachymetry determinations on both corneas showed values in all cases higher than 400 nm.

All the subjects underwent refractive surgery (LASIK or PRK) on both eyes, and the basic pharmacological treatment after the operation consisted of the administration of a corticosteroid (i.e. formocortal) in combination with an antibiotic (i.e. gentamicin) in the commercially available product Formomicin (Farmigea). The latter was administrated to the patients at a dosage of one drop of the product three times a day over a period of one month after surgery.

The contrast sensitivity was determined bilaterally for all the patients under test, both before and at fixed intervals after the operation, using a suitable instrument (Kontrastometer BA-4 from BKG Medizin Technik, Germany). The instrument allows to automatically evaluate the smallest brightness difference perceivable using a 500 level grey scale (black corresponding to 0 and white corresponding to 500, respectively), both for scotopic vision - i.e. in conditions of adaptation of the eye to darkness - and for mesopic or twilight vision - i.e. in underlighting conditions. The same apparatus allows to test the contrast after dazzling (i.e., glare), in order to examine the rate of visual re-adaptation. A two-way experimental data processing system (Anova Split-Plot) was employed for the statistical analysis of the results; while the comparison of the observed averages was made by the Tukey test.

Only the treated subjects showing, at the instrumental control carried out 7 days after the operation, a decrease of the contrast sensitivity with respect to the baseline value were considered in the clinical test.

### First stage of the study

In a first stage of the study, in order to compare the activity of the combination according to the invention with that of the formulations based on pilocarpine only or on sodium sulfacetamide only, 78 subjects were divided into three groups, each including 26 patients. Each group underwent a bilateral correction and, on the basis of the evaluation of the contrast sensitivity made at 7 days after the surgical treatment the patients suitable for the test decreased from 78 to 75. Each selected patient was treated by topical ocular administration, starting from the 7^{th} day after surgery, either with the combination of pilocarpine and sulfacetamide according to the invention, or with pilocarpine only or with sulfacetamide only, according to the following scheme:

| | combination | pilocarpine | sulfacetamide | total |
|---|---|---|---|---|
| LASIK | 11 | 14 | 12 | 37 |
| PRK | 15 | 11 | 12 | 38 |
| Total | 26 | 25 | 24 | 75 |

Each patient was treated bilaterally with two eye-drops three times a day till 60 days after surgery. Subsequent determinations of the contrast sensitivity were carried out at 15, 30, 45 and 60 days after the operation.

Furthermore, as subjective symptom, the photophobia was considered, evaluated using an arbitrary scale (absent = 0, low = +, moderate = ++, high = +++). The determinations were carried out at the same times as for the instrumental examination of the contrast sensitivity. For the statistical comparison among groups the non-parametric method of Krustal-Wallis was used.

In the following tables 4-6 the overall evaluation of the contrast sensitivity test is reported. Table 4 shows the mean threshold values determined in scotopic vision for the right eye (RE) and the left eye (LE) respectively, for each of the three groups treated with the preparations under comparison.

**TABLE 4**

| Threshold contrast* - Scotopic vision | | | | | | | |
|---|---|---|---|---|---|---|---|
| Treatment | | Baseline | 7 days | 15 days | 30 days | 45 days | 60 days |
| Combination | RE | 80.81 | 129.23 | 109.23 | 99.77 | 92-73 | 85.69 |
| | LE | 82.31 | 132.04 | 110.85 | 101.12 | 93.77 | 86.58 |
| Pilocarpine | RE | 94.24 | 121.28 | 117.72 | 114.96 | 112.52 | 109.68 |
| | LE | 92.36 | 118.16 | 114.36 | 111.56 | 109.08 | 106.56 |
| Sulfacetamide | RE | 96.54 | 135.96 | 131.54 | 127.46 | 124.33 | 120.96 |
| | LE | 97.96 | 138.00 | 132.58 | 128.38 | 125.25 | 121.46 |

| | | | | | | | |
|---|---|---|---|---|---|---|---|
| * smallest brightness difference perceivable using a scale with 500 grey levels (black = 0; white = 500) | | | | | | | |

As it is shown by the above data, in all cases the vision of the patients had become worse on the 7^{th} day after the laser operation, when the ophthalmic treatment with the drugs under test started. Thereafter, considering the trend of the contrast sensitivity over the time for each group of patients, it is possible to observe that only the patients treated with the combination according to the invention experienced a remarkable improvement of the contrast sensitivity in night vision, both for the right and for left eye.

Actually, the threshold contrast values for the right eye are significantly lower at 15 days (15.5% reduction when compared to the value at 7 days) and the improvement has continued after 30 days (22.8% reduction when compared to the value at 7 days) and also after 45 days (28.2% reduction when compared to the value at 7 days). At the end of the study (60 days) the threshold contrast value was 33.7% lower than the value determined at 7 days, while for the eyes of patients treated with pilocarpine only the final mean decrease was 9.6% of the value determined at 7 days and for those treated with sulfacetamide the reduction was 10.7% of the respective value at 7 days.

The same considerations are valid for the study of the contrast sensitivity of the left eye in each group of treated patients: for the combination according to the invention the threshold contrast at 15 days was reduced by 16% when compared to the value at 7 days, by 23.4% at 30 days, by 29% at 45 days, by 4.4% at 60 days, while for the left eye of the patients treated with pilocarpine alone the final (60 days) mean decrease was 9.8% of the value determined at 7 days and for those treated with sulfacetamide the reduction was 12%, again in comparison with the value at 7 days.

In general, it is possible to conclude that the value of the considered parameter showed limited improvements in the case of treatment with sulfacetamide or with pilocarpine alone, while the use of the proposed combination resulted in remarkable variations, evidenced by the progressive improvement of the clinical response over the time considered.

In the following Table 5 there are reported the mean values of the threshold contrast in mesopic vision, again both for the right eye (RE) and for the left eye (LE), determined for each of the three groups treated with the three preparations under comparison.

**TABLE 5**

| Threshold contrast* - Mesopic vision | | | | | | | |
|---|---|---|---|---|---|---|---|
| Treatment | | Baseline | 7 days | 15 days | 30 days | 45 days | 60 days |
| Combination | RE | 104.58 | 269.35 | 176.35 | 137.42 | 124-23 | 117.54 |
| | LE | 103.73 | 270.92 | 178.58 | 136.88 | 125.12 | 117.31 |
| Pilocarpine | RE | 104.28 | 266.52 | 249.88 | 238.68 | 225.16 | 210.12 |
| | LE | 101.04 | 257.32 | 242.04 | 233.68 | 221.04 | 204.60 |
| Sulfacetamide | RE | 107.17 | 223.42 | 207.75 | 200.63 | 189.88 | 178.71 |
| | LE | 113.16 | 243.88 | 234.08 | 227.42 | 211.04 | 197.58 |

| | | | | | | | |
|---|---|---|---|---|---|---|---|
| * smallest brightness difference perceivable using a scale with 500 grey levels (black = 0; white = 500) | | | | | | | |

Obviously, also in the case of twilight vision the contrast sensitivity for all groups of patients was remarkably reduced at 7 days after the operation, whereas for the patients treated with the combination the sensitivity had remarkably improved at 15 days. Such a trend is much less pronounced in the other two groups of treated patients. Actually, considering the right eye, the per cent variations of the sensitivity threshold from the value determined on the 7^{th} day are quite different -56.4% for the combination, -21.2% for pilocarpine, and -20% for sulfacetamide. Analogously, by comparing the values for the left eye, the following figures are obtained: -56.7% the combination, -20.5% for pilocarpine alone and -19% for sulfacetamide atone.

As a further set of comparison data, Table 6 shows the mean values obtained from the determinations of the rate of vision re-adaptation after induced dazzlement (glare) in mesopic vision, for the right and left eye, respectively, using each of the three compared treatments.

**TABLE 6**

| Contrast after induced dazzlement (glare) in mesopic vision* | | | | | | | |
|---|---|---|---|---|---|---|---|
| Treatment | | Baseline | 7 days | 15 days | 30 days | 45 days | 60 days |
| Combination | RE | 2.23 | 6.31 | 3.08 | 2.50 | 2.27 | 2.23 |
| | LE | 2.23 | 6.19 | 3.15 | 2.46 | 2.20 | 2.23 |
| Pilocarpine | RE | 2.16 | 4.68 | 3.32 | 2.60 | 2.36 | 2.16 |
| | LE | 2.00 | 4.68 | 3.00 | 2.24 | 2.12 | 2.00 |
| Sulfacetamide | RE | 2.29 | 6.21 | 4.58 | 3.79 | 3.04 | 2.63 |
| | LE | 2.42 | 5.38 | 3.92 | 3.29 | 2.71 | 2.54 |

| | | | | | | | |
|---|---|---|---|---|---|---|---|
| * indicative of the vision re-adaptation | | | | | | | |

As it is possible to see from the above table, considering the worsening induced in all patients on the 7^{th} day after photokeratectomy, in all cases an appreciable improvement of the clinical response at the 15^{th} day may be noticed. However, the per cent variations observed for the three groups of patients were different: for those treated using the combination the mean improvements were 64.7% and 64% the right and the left eye, respectively (in comparison with the values determined at 7 days), while for the patients treated with pilocarpine the mean improvements were 53.8% for the right eye and 57.3% for the left eye and, finally, for the patients treated with sulfacetamide the mean improvements were 57.6% for the right eye and 52.8% for the left eye.

As mentioned in the foregoing, in all examined patients also photophobia symptoms were detected, at the same times after the operation. From the statistical evaluation, starting from the 15^{th} day, the score of such parameter turned out to be appreciably lower in the group of patients treated using the combination than in the groups treated using sulfacetamide alone or pilocarpine alone. This better effect was maintained during the whole period of the study, till the last determination 60 days after surgical treatment.

### Second stage of the study

The second stage of the clinical study herein reported involved 32 subjects bilaterally treated by LASIK or PRK, who were not given the ocular treatments based on the examined drugs, so as to represent a control group. Obviously, the pharmacological treatment with antibiotic and corticosteroid drugs was the same as for the patients of the first stage of the study.

The same instrumental and clinical tests as for the previous groups were carried out on 32 patients, determining the baseline contrast sensitivity and the contrast sensitivity at 7, 15, 30 and 45 days after the operation.

The results obtained for this group of patients are reported in Table 7 below, limitedly to the contrast sensitivity in scotopic and mesopic vision. Contrary to the case of Tables 4-6, the values reported in Table 7 represent the averages resulting from the determinations on both the right and left eye of the examined patients.

**TABLE 7**

| Threshold contrast* - Scotopic and mesopic vision | | | | | | |
|---|---|---|---|---|---|---|
| Treatment | | Baseline | 7 days | 15 days | 30 days | 45 days |
| Control group (not treated) | Scotopic vision | 97.88 | 139.51 | 133.57 | 128.99 | 125.68 |
| | Mesopic vision | 107.81 | 230.32 | 207.91 | 200.72 | 196.21 |

| | | | | | | |
|---|---|---|---|---|---|---|
| *smallest brightness difference perceivable using a scale with 500 grey levels (black = 0; white = 500) | | | | | | |

The results reported above were statistically compared with those obtained in the first stage of the study (control group vs. treated groups). As it can be observed both in scotopic and in mesopic vision, the mean baseline value is significantly lower than the values observed after the subsequent periods, the worst response being, also in this case, on the 7^{th} day after the operation. From the 15^{th} day the contrast sensitivity naturally tends to a small improvement, quantifiable in the following terms: for the scotopic vision, -4.3% (of the value at 7 days) at 15 days, -7.5% at 30 days and -9.9% at 45 days; for the mesopic vision -9.7% (of the value at 7^{th} day) at 15 days, -12.9% at 30 days and -14.8% at 45 days.

From the above data, compared with those obtained in the first step of the study for the groups treated using only pilocarpine or only sulfacetamide, it appears that the progressive improvements in the quality of vision obtained by treating the patients with each one of the two active ingredients individually considered are not statistically different from the responses obtained in complete absence of a specific treatment. This represents a confirmation of the synergistic effect of the combination according to the invention. The latter represents the only treatment, among those examined, able to induce improvements in the contrast sensitivity more pronounced and rapid than those resulting from the normal physiological restoration of the vision functions impaired by the comeal photoablation treatment.

The present invention has been disclosed with particular reference to some specific embodiments thereof, but it should be understood that variations or modifications may be made by the person skilled in the art without departing from the scope of the invention as defined in the appended claims.

## Claims

1. An ophthalmic composition for the treatment of visual disorders **characterised by** a reduced contrast sensitivity, containing, in combination, therapeutically effective amounts of one or more miotic agents selected from cholinomimetic agents and cholinesterase inhibitors, and a hypertonic amount of one or more agents active, when applied at hypertonic concentrations, in reducing corneal oedema.

2. An ophthalmic composition according to claim 1 wherein said oedema-reducing hypertonic agent is selected from sulfacetamide and derivatives thereof, sodium chloride, glucose and glycerol.

3. An ophthalmic composition according to claim 2 wherein said miotic agent is selected from pilocarpine and pharmaceutically acceptable acid addition salts thereof, carbachol, acetylcholine, aceclidine and physostigmine.

4. An ophthalmic composition according to claims 2 or 3 wherein said hypertonic agent is sulfacetamide or a pharmaceutically acceptable salt thereof.

5. An ophthalmic composition according to claim 4 containing, in combination, pilocarpine or a pharmaceutically acceptable acid addition salt thereof end sodium sulfacetamide.

6. An ophthalmic composition according to claim 5 wherein pilocarpine or said salt thereof is present at a concentration comprised between 0.05 and 5% by weight, expressed as the free base form.

7. An ophthalmic composition according to claim 8, wherein said concentration is comprised between 0.05 and 0.5% by weight, expressed as the free base form.

8. An ophthalmic composition according to any one of claims 5-7 wherein sodium sulfacetamide is present at a concentration comprises between 5 and 30% by weight.

9. An ophthalmic composition according to claim 8 wherein the concentration of sodium sulfacetamide is comprised between 25 to 30% by weight.

10. An ophthalmic composition according to any one of the preceding claims in the form of an aqueous solution or suspension in a pharmaceutically acceptable ophthalmic vehicle.

11. The ophthalmic composition according to claim 10 containing pilocarpine hydrochloride at a concentration of 0.095% by weight (expressed as the salt form) and sodium sulfacetamide at a concentration of 28.56% by weight in aqueous solution in a pharmaceutically acceptable carrier.

12. Use of a combination of one or more miotic agents selected from cholinomimetic agents and cholinesterase inhibitors agents with a hypertonic amount of one or more agents active, when applied at hypertonic concentrations, in reducing corneal oedema, in the manufacture of a topical ophthalmic preparation for the treatment of visual disorders **characterised by** a reduced contrast sensitivity.

13. Use according to claim 12 wherein said oedema-reducing hypertonic agent is selected from sulfacetamide and derivatives thereof, sodium chloride, glucose and glycerol.

14. Use according to claim 13 wherein said combination consists of pilocarpine or a pharmaceutically acceptable acid addition salt thereof and sodium sulfacetamide.

15. Use according to claim 14 wherein the concentration of pilocarpine or of the salt thereof is comprised between 0.05 and 0.5% by weight, expressed as the free base form, and the concentration of sodium sulfacetamide is comprised between 25 and 30% by weight.

## Patentansprüche

1. Ophthalmische Zusammensetzung zur Behandlung von Augenkrankheiten, **gekennzeichnet durch** eine verminderte Kontrast-Empfinlichkeit, welche in Kombination eine therapeutisch wirksame Menge eines oder mehreren miotischen, unter den cholinomimetischen Wirkstoffen und Cholinesterase-Hemmstoffen ausgewählten Wirkstoffen, und eine hypertonische Menge eines oder mehreren Wirkstoffen, die wenn in hypertonischen Konzentrationen angewandt werden, zur Verminderung des Hornhautödems wirksam sind.

2. Ophthalmische Zusammensetzung nach Anspruch 1, worin der vorgenannte Ödem reduzierende hypertonische Wirkstoff unter den Sulfacetamiden und deren Derivaten, Natriumchlorid, Glukose und Glyzerin ausgewählt wird.

3. Ophthalmische Zusammensetzung nach Anspruch 2, worin der vorgenannte miotische Wirkstoff unter Pilokarpin und dessen pharmazeutisch verträglichen Zusatzsäuresalzen, Carbachol, Acetylcholin, Aceclidin, und Physostigmin ausgewählt wird.

4. Ophthalmische Zusammensetzung nach Anspruch 2 oder 3, worin der vorgenannte hypertonische Wirkstoff aus Sulfacetamid oder deren pharmazeutisch verträglichen Salzen besteht.

5. Ophthalmische Zusammensetzung nach Anspruch 4, die in Kombination Pilokarpin oder einen deren pharmaceutisch verträglichen Zusatzsäuresalz und Natriumsulfacetamid enthält.

6. Ophthalmische Zusammensetzung nach Anspruch 5, worin Pilokarpin oder deren vorgenannter Salz in eine Konzentration zwischen 0,05 und 5 Gew. %, ausgedrückt als freie Baseform, enthalten ist.

7. Ophthalmische Zusammensetzung nach Anspruch 6, worin die vorgenannte Konzentration zwischen 0,05 und 0,5 Gew. %, ausgedrückt als freie Baseform, liegt.

8. Ophthalmische Zusammensetzung nach je einem der Anspruche 5 bis 7, worin das Natriumsulfacetamid in einer Konzentration zwischen 5 und 30 Gew. % enthalten ist.

9. Ophtalmische Zusammensetzung nach Anspruch 8, worin die Konzentration des Natriumsulfacetamids zwischen 25 und 30 Gew.% liegt.

10. Ophthalmische Zusammensetzung nach je einem der vorhergehenden Ansprüche, unter der Form einer wässrigen Lösung oder Aufschwemmung in einem pharmazeutisch verträglichen opthalmischen Träger.

11. Ophthalmische Zusammensetzung nach Anspruch 10, welche Pilokarpinhydrochlorid in einer Konzentration von 0,095 Gew % (ausgedrückt als Salzform) und Natriumsulfacetamid in einer Konzentration von 28, 56 Gew % in wässriger Lösung in einem pharmazeutisch verträglichen Träger enthält.

12. Anwendung einer Kombination eines oder mehreren miotischen, unter den cholinomimetischen Wirkstoffen und Cholinesterase-Hemmstoffen ausgewählten Wirkstoffen, und eine hypertonische Menge eines oder mehreren Wirkstoffen, die wenn in hypertonischen Konzentrationen angewandt werden, zur Verminderung des Hornhautödems wirksam sind, in der Vorbereitung eines topischen ophthalmischen Präparats für die Behandlung von Augenkrankheiten **gekennzeichnet durch** eine verminderte Kontrast-Empfindlichkeit.

13. Anwendung nach Anspruch 12, worin der vorgenannte Ödem reduzierende hypertonische Wirkstoff unter Sulfacetamid und deren Derivaten, Natriumchlorid, Glukose und Glycerin ausgewählt wird.

14. Anwendung nach Anspruch 13, worin die vorgenannt Kombination aus Pilokarpin oder einen deren pharmazeutisch verträglichen Säurezusatzsalz und Natriumsulfacetamid besteht.

15. Anwendung nach Anspruch 14, worin die Konzentration von Pilokarpin oder deren Salzes zwischen 0,05 und 0,5 Gew %, ausgedrükt als die freie Baseform, und die Konzentration des Natriumsulfacetamids zwischen 25 und 30 Gew % liegt.

## Revendications

1. Composition ophtalmique pour le traitement des défauts de la vue **caractérisés par** une sensibilité au contraste réduite, comprenant, en combination, des quantités pharmaceutiquement efficaces d'un ou de plusieurs agents myotiques, choisis entre les agents cholinomimétiques et les inhibiteurs de cholinestérase, et une quantité hypertonique d'un ou de plusieurs agents actives, si appliqués en concentrations hypertoniques, pour réduir l'oedème cornéen.

2. Composition ophtalmique selon la revendication 1, dans laquelle ledit agent hypertonique de réduction d'oedème est choisi entre sulfacétamide et ses dérivés, chlorure de sodium, glucose et glycérol .

3. Composition ophtalmique selon la revendication 2, dans laquelle ledit agent myotique est choisi entre: pilocarpine et ses sels d'addition acide pharmaceutiquement acceptables, carbachol, acétylcholine, acéclidine et physostigmine.

4. Composition ophtalmique selon les revendications 2 ou 3, dans laquelle ledit agent hypertonique est sulfacétamide ou un de ses sels pharmaceutiquement acceptables.

5. Composition ophtalmique selon la revendication 4, contenant, en combination, pilocarpine ou un de ses sels d'addition acide et sulfacétamide sodique.

6. Composition ophtalmique selon la revendication 5, dans lequelle ladite pilocarpine ou son sel est présente en une concentration entre 0,05 et 5% en poids, exprimée comme la forme de base libre.

7. Composition ophtalmique selon la revendication 6, dans laquelle ladite concentration est comprise entre 0,05 et 0,5% en poids, exprimée comme la forme de base libre.

8. Composition ophtalmique selon une quelconque des revendications 5 à 7, dans laquelle le sulfacétamide sodium est présent en une concentration cmprise entre 5 et 30% en poids.

9. Composition ophtalmique selon la revendication 8, dans laquelle la concentration du sulfacétamide sodique est comprise entre 25 et 30% en poids.

10. Composition ophtalmique selon une quelconque des revendications précédentes, en forme d'une solution ou suspension aqueuse dans un véhicule ophtalmique pharmaceutiquement acceptable.

11. Composition ophtalmique selon la revendication 10, contenant hydrochlorure de pilocarpine en une concentration de 0,095% en poids (exprimée comme la forme de sel) et de sulfacétamide sodique en une concentration de 28,56% en poids en solution aqueuse dans un véhicule pharmaceutiquement acceptable.

12. Utilisation d'une combination d'un ou des plusieurs agents myotiques choisis entre les agents cholinomimétiques et les agents inhibiteurs de cholinestérase avec une quantité hypertonique d'un ou de plusieurs agents actives, si appliqués en concentrations hypertoniques, pour réduire l'oedème cornéen, dans la préparation d'un médicament ophtalmique topique pour le traitement des défauts de la vue **caractérisés par** une sensibilité au contraste réduite.

13. Utilisation selon la revendication 12, dans laquelle ledit agent hypertonique de réduction d'oedème est choisi entre sulfacétamide et ses dérivés, chlorure de sodium, glucose et glycerol.

14. Utilisation selon la revendication 13, dans laquelle ladite combination consiste de pilocarpine ou d'un de ses sels d'addition acide pharmaceutiquement aceptable et de sulfacétamide sodique.

15. Utilisation selon la revendication 14, dans laquelle la concentration de pilocarpine ou d'un des ses sels d'addition acide est comprise entre 0,05 et 0,5% en poids, exprimée comme la forme de base libre, et la concentration de sulfacétamide est comprise entre 25 et 30% en poids.
